(19) Europäisches Patentamt
European Patent Office
Office européen des brevets

(11) Veröffentlichungsnummer: **0 243 308 B1**

(12) **EUROPÄISCHE PATENTSCHRIFT**

(45) Veröffentlichungstag der Patentschrift: **15.05.91**

(51) Int. Cl.$^5$: **A61K 47/00**

(21) Anmeldenummer: **87810219.3**

(22) Anmeldetag: **08.04.87**

(54) **Stabilisierung des Konservierungsmittels Thiomersal in Augenarzneimitteln.**

(30) Priorität: **14.04.86 DE 3612538**

(43) Veröffentlichungstag der Anmeldung:
**28.10.87 Patentblatt 87/44**

(45) Bekanntmachung des Hinweises auf die Patenterteilung:
**15.05.91 Patentblatt 91/20**

(84) Benannte Vertragsstaaten:
**BE CH FR GB IT LI LU NL SE**

(56) Entgegenhaltungen:
**GB-A- 1 173 661**
**US-A- 2 958 625**
**US-A- 4 524 063**

(73) Patentinhaber: **DISPERSA AG**
**Riethofstrasse 1**
**CH-8442 Hettlingen(CH)**

(72) Erfinder: **Doulakas, Johann, Dr.**
**Landenbergstrasse 33**
**CH-8404 Winterthur(CH)**

(74) Vertreter: **Schluep, Hans-Peter et al**
**c/o CIBA GEIGY AG Patentabteilung Postfach**
**CH-4002 Basel(CH)**

**EP 0 243 308 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist die Stabilisierung des quecksilberhaltigen Konservierungsmittels Thiomersal in Augenarzneimitteln, z.B. Augentropfen oder Augengelen, durch den Zusatz von 2-Amino-2-hydroxymethyl-1,3-propandiol (Trometamol) oder einem Homologen davon mit bis zu 10 C-Atomen. Durch die Erfindung wird eine erhöhte Stabilität von Thiomersal enthaltenden Augenarzneimitteln sowie eine verbesserte Verträglichkeit dieser Präparate im Auge erreicht.

Arzneimittel zur Behandlung von Erkrankungen im Auge werden häufig in flüssiger Form formuliert und in Tropfenform verabreicht. Bei Augentropfen ist in den meisten europäischen und anderen Ländern eine Konservierung der Präparate vorgeschrieben, d.h. ein Schutz gegen einen Befall und eine anschliessende Vermehrung von Mikroorganismen durch den Zusatz einer Komponente, die in der Lage ist, eine sekundäre Kontamination zu verhindern oder sie zumindest einzudämmen. Weiterhin werden Augenarzneimittel häufig als Gel formuliert. Für Gele gilt das gleiche bezüglich Konservierung.

Als besonders gut geeignete Konservierungsmittel haben sich für die Verwendung in Augenarzneimitteln bestimmte quecksilberhaltige Stoffe erwiesen, insbesondere das Natriumsalz von 2-(Aethylmercurithio)-benzoesäure (Thiomersal).

Thiomersal hat jedoch verschiedene Nachteile. Beispielsweise lagert es sich im Lauf der Zeit an der Oberfläche der Innenseite der Kunststoffbehälter ab, in denen Augenarzneimittel üblicherweise aufbewahrt werden. So nimmt der Gehalt an Thiomersal in Kunststoff-Fläschchen aus Niederdruckpolyäthylen bereits innerhalb von wenigen Wochen auf unter etwa 80 % des ursprünglichen Gehalts ab und nach 1/2 bis 1 Jahr befindet sich praktisch kein oder nur mehr eine sehr geringe Menge Thiomersal in der Augentropfen- bzw. Augengel-Formulierung.

Ein weiterer Nachteil von Thiomersal ist seine geringe Stabilität in wässriger Lösung. Es wird wegen seiner hervorragenden Konservierungseigenschaften besonders häufig verwendete, ist aber in wässriger Lösung unstabil.

Der Erfindung liegt somit die Aufgabe zugrunde, ein als Konservierungsmittel Thiomersal enthaltendes flüssiges oder gelförmiges Augenarzneimittel zu schaffen, das ausgezeichnete Stabilität für einen ausreichenden Zeitraum von Lagerung und Benutzung aufweist. Diese Aufgabe wird durch den überraschenden Befund gelöst, dass durch den Zusatz von bestimmten Aminopolyolen eine Stabilisierung von Thiomersal in flüssigen Augenarzneimitteln erreicht werden kann.

Gegenstand der Erfindung ist somit ein als Konservierungsmittel Thiomersal enthaltendes, in Tropfenform oder Gelform zu verabreichendes Augenarzneimittel, das dadurch gekennzeichnet ist, dass es zur Stabilisierung des Konservierungsmittels 2-Amino-2-hydroxymethyl-1,3-propandiol oder ein Homologes davon mit bis zu 10 C-Atomen in einer Menge von 0,1 bis 1 Gewichtsprozent enthält.

Gegenstand der Erfindung ist auch die Verwendung von 2-Amino-2-hydroxymethyl-1,3-propandiol oder einem Homologen davon mit bis zu 10 C-Atomen zur Stabilisierung des Konservierungsmittels Thiomersal in Augenarzneimitteln.

Das 2-Amino-2-hydroxymethyl-1,3-propandiol, das unter verschiedenen Bezeichungen bekannt ist [Tromethamin, Tris(hydroxymethyl)-aminomethan, Tris, THAM, Trisamin, Tromethan; internationaler Freiname: Trometamol] hat die folgende Formel:

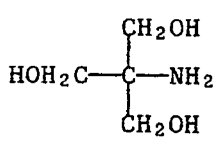

Summenformel: $C_4H_{11}NO_3$ Mol.-Gew.: 121,14

Trometamol wird als Standard in der Massanalyse sowie als Puffer für mikrobiologische und pharmazeutische Zwecke verwendet. Ferner wird es in der Kosmetik als Emulgat or und in der Chemie, insbesondere der pharmazeutischen Chemie, als Lösungsvermittler eingesetzt. Ausserdem wird es in der Medizin gegen Acidosis in Form von intravenösen Injektionen verabreicht.

Die Homologen von 2-Amino-2-hydroxymethyl-1,3-propandiol, die für die Zwecke der Erfindung brauchbar sind, können bis zu 10 C-Atome aufweisen. Diese homologen Verbindungen ergeben sich durch den Einbau weiterer Kohlenstoffatome an einer oder mehreren Stellen des vorstehend gezeigten Molekülgerüstes. Bevorzugt sind die Homologen mit 5 bis 7 C-Atomen.

Trometamol und seine Homologen mit bis zu 10, bevorzugt 5 bis 7, C-Atomen lassen sich auch durch

die folgende Formel veranschaulichen:

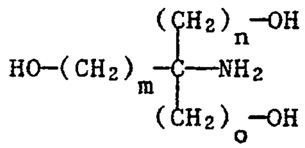

m, n, o (unabhängig voneinander) ≙ eine ganze Zahl, mindestens 1
Summe aus m, n und o ≙ 3 bis 9, bevorzugt 3 bis 6

Durch den Zusatz des erfindungsgemäss vorgeschlagenen Stabilisators wird das Thiomersal in flüssigen oder gelförmigen Augenheilmitteln wirksam stabilisiert. Es hat sich gezeigt, dass das Trometamol bzw. seine Homologen mit bis zu 10 C-Atomen die Ablagerung von Thiomersal auf der Oberfläche der Kunststoffbehälter verhindern. Thiomersal bleibt bei Zusatz des erfindungsgemäss vorgeschlagenen Stabilisators für mehrere Jahre in Lösung.

Der erfindungsgemäss vorgeschlagene Stabilisator hat den weiteren Vorteil, dass er die Neigung von Thiomersal zur Zersetzung in wässriger Lösung unterdrückt. Thiomersal, ist in wässriger Lösung nicht über längere Zeit stabil. Die Stabilisierung wird vor allem dadurch erreicht, dass die Ad- bzw. Absorption von Thiomersal an der Innenwand der Kunststoffbehälter verhindert bzw. verlangsamt wird. Durch den Zusatz von Trometamol kann die chemische Zersetzung des Thiomersal in der wässrigen Augenformulierung für längere Zeit unterdrückt werden, so dass das Konservierungsmittel über den gesamten Zeitraum der üblichen Lager- und Gebrauchszeit des Präparates zur Verfügung steht. Der erfindungsgemäss eingesetzte Stabilisator verhindert ferner auch die Ablagerung von gegebenenfalls entstandenen Spaltprodukten des Thiomersals, wie Aethylquecksilbersalzen, an der Wand von Kunststoffbehältern.

Ueberraschenderweise hat sich ausserdem gezeigt, dass durch den Zusatz des erfindungsgemäss vorgeschlagenen Stabilisators die Verträglichkeit bestimmter Augenarzneimittel im Auge verbessert wird. Dies trifft beispielsweise für ein jüngst entwickeltes Präparat zur Behandlung stärkerer akuter oder chronisch rezidivierender Entzündungserscheinungen am Auge zu, das Diclofenac-Natrium als nichtsteroidalen Entzündungshemmer enthält (vgl. nachstehendes Beispiel 1). Bei diesem Präparat, das Thiomersal als Konservierungsmittel enthält, führt der Zusatz von Trometamol zu einer deutlichen Verbesserung der Verträglichkeit im Auge.

Der erfindungsgemäss vorgeschlagene Stabilisator wird den flüssigen oder gelförmigen Augenarzneimitteln in einer Menge von 0,1 bis 1,0 % zugesetzt. In dieser Menge verhindert der Stabilisator die Ablagerung von Thiomersal an der Innenwand der Kunststoffbehälter und bewirkt so seine gewünschte Stabilisierung. Damit kann Thiomersal die erwünschte Wirkung gegen Befall und Wachstum von Mikroorganismen über längere Zeit entfalten. Die Präparate können bei Raumtemperatur gelagert werden und erfüllen die Anforderungen, die während des therapeutischen Gebrauchs hinsichtlich ihrer Haltbarkeit gestellt werden.

Das Augenarzneimittel kann jeden zur Anwendung im Auge geeigneten Wirkstoff, oder Mischungen von mehreren Wirkstoffen, enthalten. Neben den in den Beispielen genannten Wirkstoffen sind besonders zu erwähnen: Spagluminsäure [N-(N'-Acetyl-L-$\beta$-aspartyl)-L-glutaminsäure] und Gentamycin.

Die folgenden Beispiele erläutern die Erfindung:

Beispiel 1: Es wird ein Augenarzneimittel zur Behandlung von Entzündungen hergestellt, das Diclofenac-Natrium als nichtsteroidalen Entzündungshemmer enthält. Als Konservierungsmittel wird Thiomersal und als Stabilisator Trometamol verwendet. Die Formulierung weist folgende Zusammensetzung auf:

| Bestandteil | Menge |
|---|---|
| Diclofenac-Natrium | 0,1 % |
| 2-(Aethylmercurithio)-benzoesäure, Natriumsalz (Thiomersal) | 0,004 % |
| Borsäure | 1,9 % |
| Trometamol (Stabilisator) | 0,6 % |
| Cremophor EL® (Reaktionsprodukt aus Rizinusöl und Aethylenoxid) (Lösungsvermittler) | 5,0 % |
| Wasser für Injektionszwecke | Restmenge |

Diese Formulierung ist bei Raumtemperatur 3 bis 5 Jahre haltbar, ohne dass in diesem Zeitraum eine wesentliche Abnahme des Gehalts an Konservierungsmittel festgestellt wird.

Beispiel 2:

| Bestandteil | Menge |
|---|---|
| Chloramphenicol | 0,600 g |
| Polyethylenglykol 400 | 10,000 g |
| Borsäure | 0,900 g |
| Trometamol | 0,100 g |
| 2-(Aethylmercurithio)-benzoesäure, Natriumsalz | 0,020 g |
| Hydroxypropylmethylcellulose | 0,200 g |
| Prednisolonacetat | 0,500 g |
| Aluminiumhydroxid-Gel | 1,000 g |
| Wasser für Injektionszwecke | 88,780 g |
| | 102,100 g ≙ 100 ml |

Die Formulierung des Beispiels 2 weist eine ähnlich lange Haltbarkeit ohne nennenswerte Abnahme des Gehalts an Konservierungsmittel auf wie die Formulierung von Beispiel 1.

## Ansprüche

1. Ein als Konservierungsmittel Thiomersal enthaltendes, in Tropfenform oder Gelform zu verabreichendes Augenarzneimittel, dadurch gekennzeichnet, dass es zur Stabilisierung des Konservierungsmittels 2-Amino-2-hydroxymethyl-1,3-propandiol oder ein Homologes davon mit bis zu 10 C-Atomen in einer Menge von 0,1 bis 1 Gewichtsprozent enthält.

2. Augenarzneimittel nach Anspruch 1, dadurch gekennzeichnet, dass es zur Stabilisierung des Konservierungsmittels 2-Amino-2-hydroxymethyl-1,3-propandiol oder ein Homologes davon mit 5 bis 7 C-Atomen enthält.

3. Augenarzneimittel nach Anspruch 1, dadurch gekennzeichnet, dass es zur Stabilisierung des Konservierungsmittels 2-Amino-2-hydroxymethyl-1,3-propandiol enthält.

4. Augenarzneimittel nach einem der Ansprüche 1 bis 3, dadurch gekennzeichnet, dass es sich um Augentropfen handelt.

5. Verwendung von 2-Amino-2-hydroxymethyl-1,3-propandiol oder einem Homologen davon mit bis zu 10 C-Atomen bei der Herstellung von Augentropfen oder Augengelen, die Thiomersal als Konservierungsmittel enthalten.

6. Verwendung von 2-Amino-2-hydroxymethyl-1,3-propandiol oder einem Homologen davon mit bis zu 10 C-Atomen bei der Herstellung von Augentropfen, die Thiomersal als Konservierungsmittel enthalten.

7. Verwendung nach einem der Ansprüche 5 oder 6, dadurch gekennzeichnet, dass 2-Amino-2-hydroxymethyl-1,3-propandiol verwendet wird.

8. Verwendung von 2-Amino-2-hydroxymethyl-1,3-propandiol oder einem Homologen davon mit bis zu 10 C-Atomen zur Stabilisierung des Konservierungsmittels Thiomersal in Augentropfen oder Augengelen.

9. Verwendung von 2-Amino-2-hydroxymethyl-1,3-propandiol oder einem Homologen davon mit bis zu 10 C-Atomen zur Stabilisierung des Konservierungsmittels Thiomersal in Augentropfen.

10. Verwendung nach einem der Ansprüche 8 oder 9, dadurch gekennzeichnet, dass 2-Amino-2-hydroxymethyl-1,3-propandiol verwendet wird.

**Claims**

1. An ophthalmic medicament that contains thiomersal as preservative and is to be administered in drop form or gel form, characterized in that it contains 2-amino-2-hydroxymethyl-1,3-propanediol or a homologue thereof having up to 10 carbon atoms in an amount of from 0.1 to 1 % by weight for the stabilisation of the preservative.

2. Ophthalmic medicament according to claim 1, characterised in that it contains 2-amino-2-hydroxymethyl-1,3-propanediol or a homologue thereof having from 5 to 7 carbon atoms for the stabilisation of the preservative.

3. Ophthalmic medicament according to claim 1, characterized in that it contains 2-amino-2-hydroxymethyl-1,3-propanediol for the stabilisation of the preservative.

4. Ophthalmic medicament according to any one of claims 1 to 3, characterised in that it is in the form of eye drops.

5. Use of 2-amino-2-hydroxymethyl-1,3-propanediol or a homologue thereof having up to 10 carbon atoms in the manufacture of eye drops or eye gels that contain thiomersal as preservative.

6. Use of 2-amino-2-hydroxymethyl-1,3-propanediol or a homologue thereof having up to 10 carbon atoms in the manufacture of eye drops that contain thiomersal as preservative.

7. Use according to claim 5 or claim 6, characterised in that 2-amino-2-hydroxymethyl-1,3-propanediol is used.

8. Use of 2-amino-2-hydroxymethyl-1,3-propanediol or a homologue thereof having up to 10 carbon atoms for the stabilisation of the preservative thiomersal in eye drops or eye gels.

9. Use of 2-amino-2-hydroxymethyl-1,3-propanediol or a homologue thereof having up to 10 carbon atoms for the stabilisation of the preservative thiomersal in eye drops.

10. Use according to claim 8 or claim 9, characterised in that 2-amino-2-hydroxymethyl-1,3-propanediol is used.

## Revendications

1. Un médicament ophtalmique à administrer sous forme de gouttes ou de gel, contenant du Thiomersal en tant que conservateur, caractérisé en ce qu'il contient, pour la stabilisation du conservateur, du 2-amino-2-hydroxyméthyl-1,3-propane-diol ou un homologue de ce diol contenant jusqu'à 10 atomes de carbone, en quantité de 0,1 à 1% en poids.

2. Médicament ophtalmique selon la revendication 1, caractérisé en ce qu'il contient pour la stabilisation du conservateur du 2-amino-2-hydroxyméthyl-1,3-propane-diol ou un homologue de ce diol contenant 5 à 7 atomes de carbone.

3. Médicament ophtalmique selon la revendication 1, caractérisé en ce qu'il contient pour la stabilisation du conservateur du 2-amino-2-hydroxyméthyl-1,3-propane-diol.

4. Médicament selon l'une des revendications 1 à 3, caractérisé en ce qu'il est sous la forme de gouttes ophtalmiques.

5. Utilisation du 2-amino-2-hydroxyméthyl-1,3-propane-diol ou d'un homologue de ce diol contenant jusqu'à 10 atomes de carbone à la préparation de gouttes ophtalmiques ou gels ophtalmiques contenant du Thiomersal en tant que conservateur.

6. Utilisation du 2-amino-2-hydroxyméthyl-1,3-propane-diol ou d'un homologue de ce diol contenant jusqu'à 10 atomes de carbone à la préparation de gouttes ophtalmiques contenant du Thiomersal en tant que conservateur.

7. Utilisation selon l'une des revendications 5 ou 6, caractérisée en ce que l'on utilise du 2-amino-2-hydroxyméthyl-1,3-propane-diol.

8. Utilisation du 2-amino-2-hydroxyméthyl-1,3-propane-diol ou d'un homologue de ce diol contenant jusqu'à 10 atomes de carbone pour la stabilisation du conservateur Thiomersal dans des gouttes ophtalmiques ou des gels ophtalmiques.

9. Utilisation du 2-amino-2-hydroxyméthyl-1,3-propane-diol ou d'un homologue de ce diol contenant jusqu'à 10 atomes de carbone pous la stabilisation du conservateur Thiomersal dans des gouttes ophtalmiques.

10. Utilisation selon l'une des revendications 8 ou 9, caractérisée en ce que l'on utilise le 2-amino-2-hydroxyméthyl-1,3-propane-diol.